# EUROPEAN PATENT APPLICATION

(11) **EP 2 233 556 A1**
(43) Date of publication of application: **29.09.2010**
(21) Application number: 10003030.3
(22) Date of filing: 23.03.2010
(51) Int. Cl.: C11B 9/00, C07C 45/54, C07C 45/62, C07C 45/82, C07C 47/228, C07C 45/74

(54) **Isomeric mixture of meta and para tertio-butylphenyl propanal and production thereof**

(30) Priority: 23.03.2009 US 162646 P
(71) Applicant: O'Laughlin Industries, Co. Ltd., 34-37 Connaught Road Central Hong Kong (HK)
(72) Inventor: Zhiguo, Gao, Xinqing District Tianjin, 300382 (CN); Jun, Li, Xinqing District Tianjin, 300382 (CN); Xin, Qi, Xinqing District Tianjin, 300382 (CN)
(74) Representative: van Walstijn, Bartholomeus G. G.

(57) **Abstract**

A product of p-tert-butylphenyl propionaldehyde and m-tert-butylphenyl propionaldehyde, wherein m-tert-butylphenyl propionaldehyde is in an amount from 6% to 60% of the combination of p-tert-butylphenyl propionaldehyde and m-tert-butylphenyl propionaldehyde. Also a method including; adding dropwise an aldehyde solution to a basic solution; and, collecting a fraction of p-tert-butyl cinnamic aldehyde m-tert-butyl cinnamic aldehyde therefrom; and, adding a transition metal catalyst to a solution of the p-tert-butyl cinnamic aldehyde in ethanol, and, collecting therefrom by distillation a product ofp-tert-butylphenyl propionaldehyde and the meta isomer m-tert-butylphenyl propionaldehyde; wherein the product comprises m-tert-butylphenyl propionaldehyde in an amount from 6% to 60% of the combination of p-tert-butylphenyl propionaldehyde and m-tert-butylphenyl propionaldehyde.

## Description

### INTRODUCTION

The present disclosure is directed to aromatic propanal production methods generally, and more particularly to the production of either the compound known by its commercial name of bourgeonal thereof. Bourgeonal is known mainly as para-tertiary-butylphenyl propionaldehyde however, in some instances it may be that it may have, refer to, be related to or include a meta isomer; thus, there often may be one or two compounds of interest; namely, p-t-butylphenyl propionaldehyde and the meta isomer, m-t-butylphenyl propionaldehyde. The first, p-t-butylphenyl propionaldehyde, is shown below as Structure I, and the meta isomer, m-t-butylphenyl propionaldehyde is shown below as Structure II: 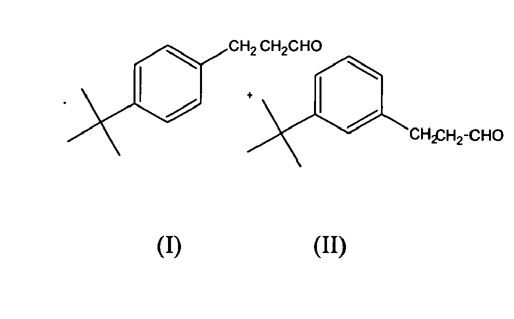 Bourgeonal is also known, particularly in the Structure (I) embodiment, as p-tert-butyldihydrocinnamic aldehyde, 3-(4-tert-butylphenyl)propanal, by its IUPAC name of 3-(4-tert-butylphenyl)-propanal, and by its INCI name 3-(4-tert-butylphenyl)propionaldehyde.

### BACKGROUND

The resulting Structures (I) and (II), have a green lily odor and have been used in soap and cosmetic perfume as perfuming agent and/or fixative.

Prior methods have been used to obtain structure (I), para-tertiary-butylhydrocinnamic aldehyde; one such method of synthesis has typically been by Friedel-Crafts reaction using t-butyl benzene (TBB) and acrolein diacetic acid ester, then hydrolysis. 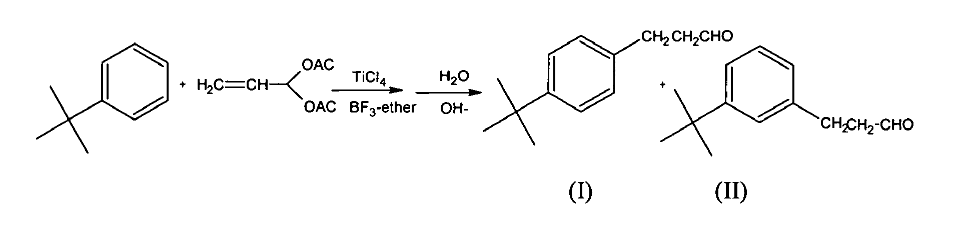 In this process, Structure (II) 3-(3-tert-butylpenyl) propionaldehyde is also obtained in a yield of about 3-6%.

A first drawback is a low yield of the desired product. However, a larger downside of such processing is that a large amount of waste water is created, generally too much, which can cause a heavy pressure on the environment. Currently, environmental concerns are paramount, so efforts have been made to develop new synthetic routes that eliminate or substantially reduce waste water pollution.

### SUMMARY

The present disclosure includes a product of p-tert-butylphenyl propionaldehyde and m-tert-butylphenyl propionaldehyde, including m-tert-butylphenyl propionaldehyde in an amount from 6% to 60% of the combination of p-tert-butylphenyl propionaldehyde and m-tert-butylphenyl propionaldehyde.

Also included is a method for the production of p-tert-butylphenyl propionaldehyde and the meta isomer m-tert-butylphenyl propionaldehyde, the method including: adding dropwise an aldehyde solution to a basic solution; and, collecting a fraction of p-tert-butyl cinnamic aldehyde m-tert-butyl cinnamic aldehyde therefrom; and, adding a transition metal catalyst to a solution of the p-tert-butyl cinnamic aldehyde in ethanol, and, collecting therefrom by distillation a product of p-tert-butylphenyl propionaldehyde and the meta isomer m-tert-butylphenyl propionaldehyde; wherein the product comprises m-tert-butylphenyl propionaldehyde in an amount from 6% to 60% of the combination of p-tert-butylphenyl propionaldehyde and m-tert-butylphenyl propionaldehyde.

The foregoing specific aspects and advantages of the present developments are illustrative of those which can be achieved by these developments and are not intended to be exhaustive or limiting of the possible advantages which can be realized. Thus, those and other aspects and advantages of these developments will be apparent from the description herein or can be learned from practicing the disclosure hereof, both as embodied herein or as modified in view of any variations which may be apparent to those skilled in the art. Thus, in addition to the exemplary aspects and embodiments described above, further aspects and embodiments will become apparent by reference to and by study of the following descriptions.

### DETAILED DESCRIPTION

The present development provides preparative methods for structures (I) and (II) that provides much higher than conventional yields and provides much less, i.e., little or substantially no pollutant discharge. Instead of using the typical TBB method described hereinabove, the methods hereof use p-t-butylbenzaldehyde, in a generally described two or three step reaction involving oxidation, aldol condensation and hydrogenation.

More particularly, the disclosure hereof is directed to one or more methods for the production of bourgeonal or an isomer thereof, e.g., the meta isomer, the methods including adding dropwise an aldehyde solution to a p-t-butylbenzaldehyde basic solution; and, collecting a fraction of p-tert-butyl cinnamic aldehyde therefrom; and, adding a transition metal catalyst to a solution of the p-tert-butyl cinnamic aldehyde in ethanol, adding hydrogen and, collecting therefrom by distillation a product of Structures (I) and (II). The resulting product may also be known generally as a variant of "bourgeonal".

The Structures (I) and (II) have an intense herb lagotis and green lily odor, especially as found through the developments hereof in the m-isomer product (about 6 to about 60% of the overall product), and the aroma has qualities that have been described as tenderness, clarity, elegance and permanence. It has been used in soap and cosmetic perfume as perfuming agent and/or fixative.

As introduced, the methods hereof minimally involve aldol condensation and hydrogenation, and generally the raw material a t-butyltoluene which contains an m-isomer. The methods hereof may increase the amount of the isomer to a higher level than previously realized, e.g., from about 5% to about 55%. It has been found that the aroma of the resultant product may be a perfume which is more robust with a higher quantity of the meta isomer or m-isomer of Structure (II).

More particularly, the methods hereof use, in lieu of TBB in the TBB predecessor process, p-t-butyltoluene as a precursor, in a reaction involving aldol condensation and hydrogenation. The processes hereof generally include aldol condensation of aryl-aldehyde (Claisen-Schmidt reaction) and an α,β-unsaturated aldehyde (cinnamaldehyde) catalytic hydrogenation reaction. A transition metal catalyst, such as nickel (Ni) or palladium (Pd) can be used in the latter reaction.

A more detailed explanation of a synthesis procedure hereof may include isomerization, oxidation, aldol condensation and hydrogenation. From p-t-butyltoluene, the route may be shown as follows:

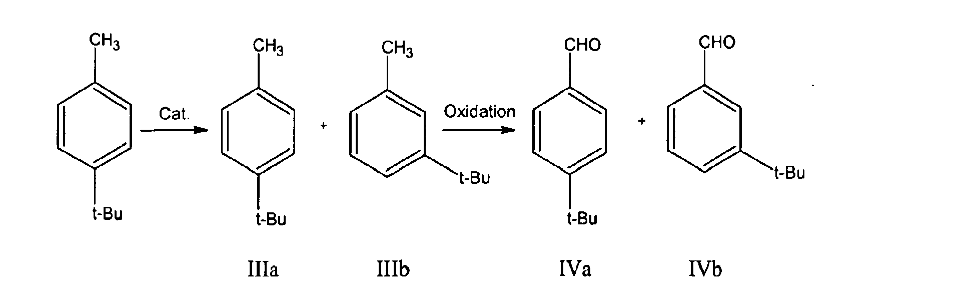

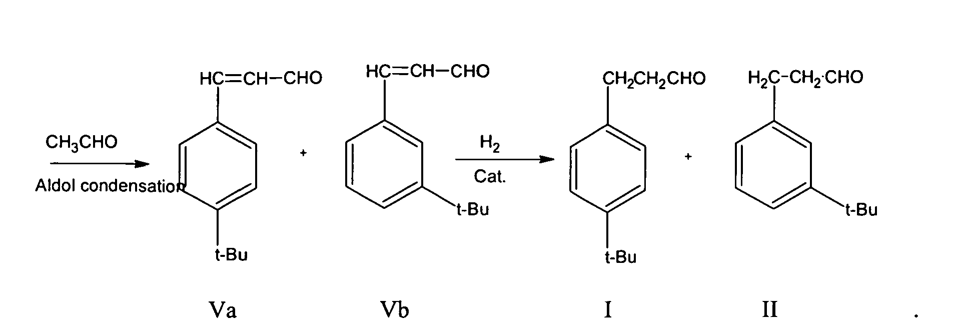

The first step as shown is the isomerization of p-t-butyltoluene via Friedel-Crafts reaction. The catalyst for this reaction may be a proton acid, such as sulphatic acid, or a Lewis acid, such as aluminium chloride, ferric chloride, or the like. The amount of Structure IIIb (m-t-butyltoluene) can be maintained at 30-60% through this step.

The second step is the oxidation of Structures IIIa and IIIb, to yield Structure IVa, p-t-butylphenyl benzaldehyde, and Structure IVb, m-t-butylphenyl benzaldehyde.

The third step is aldol condensation via Claisen-Schmidt reaction of the aromatic aldehyde Structures IVa and IVb with acetaldehyde. Generally, Claisen-Schmidt reactions involve the use of a base or basic catalyst, such as caustic alkali, i.e., sodium hydroxide or potassium hydroxide, respectively, NaOH and KOH. Also useful may be Na₂CO₃ and K₂CO₃; or the catalyst for this reaction may also be zinc oxide, ZnO, the latter being effective. The base concentration can be about 1% to about 20%, although between about 3% to about 10% appears preferable. The reaction can be in ethanol, in water, or in an ethanol water solution, ethanol water solution is preferable. The temperature can be between about 0°C and about 80°C, but between about 30°C and about 50°C is better.

In the fourth step, a catalytic hydrogenation reaction is used to provide the product structures (I) and (II). The choice of better transition metal catalyst should take into account that it is desirable that the carbon, carbon double bond should be reduced selectively to avoid the reduction of carbonyl during the catalytic hydrogenation reaction. In this step, a nickel catalyst, such as Raney nickel, a palladium catalyst, such as Pd/C, or an alloy catalyst, such as Pd-Ni composite catalysts may be used, the latter being nicely effective.

It also appears that ethanol may be more effective than other kinds of alcohol solutions, such as carbinol, or hydrocarbons, such as, for example, hexane, heptane, and cyclohexane, which could also or alternatively be used in the reaction. The hydrogenation reaction may be set to occur within the range of about 0°C and about 100°C, but it appears that the range of about 5°C to about 40°C is preferable. Similarly, this hydrogenation reaction can be within about 0.05MPa to about 3.00MPa, although between about 0.1MPa and about 1.0MPa is preferable.

The following examples may be utilized for further explaining the present developments.

### Example 1

### Isomerization of p-t-butyltoluene

In a 500ml three-neck flask, which may include a stirrer, thermometer and condenser, p-t-butyltoluene (about 100ml), toluene (about 50ml) and ferric chloride (about 1g) are added. The mixture may be agitated an additional about 0.5 hours at room temperature. The product may be checked by GC (gas chromatography), and the conversion of a substantial portion to meta isomer reached is about 30% to about 60%, that is, the content of m-tert-butyltoluene is about 30 to about 60%. Then, the mixture may be washed by about 100ml water, and the organic phase may be collected. The toluene may be removed by distillation, and the fraction is collected at 196-205°C. This fraction part is mainly Structures IIIa and IIIb. The yield is about ≥90% of the combination of structures IIIa and IIIb.

### Example 2

### Aldol condensation (Claisen-Schmidt reaction)

In a 200ml autoclave, about 100g the mixture of p-tert- and m-tertbutylbenzaldehyde (Structures IVa and IVb, oxidized resulting products obtained from the structures IIIa and IIIb of Example 1), about 10g acetaldehyde and about 1g zinc oxide were added. The mixture was stirred an additional about 1.5 hours at about 130-180°C. The product is checked by GC, and the conversion is reached about 19%. The catalyst (ZnO) may be removed by filtration, and the filtrate may be isolated by vacuum distillation. Structures IVa and IVb are recovered, and the products t-butylcinnamic aldehyde (Structures Va and Vb) are collected at about 120-140°C and about 20Pa. The yield is about 70% for Structures Va and Vb, and recovering yield of Structures IVa and IVb is 90%

### Example 3

### Aldol condensation (Claisen-Schmidt reaction) alternative

In a 500ml four-neck flask, which may include a stirrer, a condenser, a dropping funnel and a thermometer, about 100g of the mixture of Structures IVa and IVb, about 50g alcohol, about 109 sodium hydroxide and about 100g water were added. The acetaldehyde, about 10g, may be added dropwise over the course of about 1 hour at room temperature, and the mixture may be stirred for about 1 hour under the same conditions. The alcohol, which is in the mix, may be removed by distillation. Then the residue may be neutralized by about 10% hydrochloride acid to about pH 7-8, the organic phase may be isolated then washed by about 50ml water. The product may be isolated by vacuum distillation in the same manner as given in example 2. The yield may be about 65%, and the recovering yield of IVa and IVb is about 90%

### Example 4

### Catalyzed hydrogenation

In an about 200ml autoclave, about 30g of the mixture of p-tert- or m-tert-butylcinnamic aldehyde (Structures Va and Vb), about 30g Pd/C and about 60g alcohol were added. The space of the autoclave was washed with hydrogen about three times. The pressure of the hydrogen is adjusted to about 2.0MPa. The mixture may be stirred for about 8 hours at about 50°C. The product may be checked by GC, and the conversion reached is about 90%. The catalyst (Pd/C) may be recovered by filtration. The products of Structure I and Structure II are collected by vacuum distillation at about 100-120°C and about 20Pa. The yield is about 80%.

### Example 5

### Catalyzed hydrogenation alternative

The process is as same as example 4, but the catalyst is changed to a Pd-Ni composite. The yield is also about 80%, and the purity is about ≥85%.

The resulting product, Structures (I) and (II), p-tert-butylphenyl propionaldehyde, Structure (I), and m-tert-butylphenyl propionaldehyde, Structure (II), the product comprising m-tert-butylphenyl propionaldehyde in an amount from 6% to 60% of the combination of p-tert-butylphenyl propionaldehyde and m-tert-butylphenyl propionaldehyde. In many instances the resulting product is in the range of between about 50% and about 55% of structure II to structure I. The combination product presents as a colorless or pale yellow liquid. The combination product provides an intense herb lagotis and green lily odor, especially with the higher concentration or quantity of the m-isomer product relative to the para product (structure I), and the aroma has qualities that have been described as tenderness, clarity, elegance and permanence. The product may thus be used in soap and cosmetic perfume as perfuming agent and/or fixative, inter alia.

While a number of exemplary aspects and embodiments have been discussed above, those of skill in the art will recognize certain modifications, permutations, additions and sub combinations thereof. It is therefore intended that the following appended claims and claims hereafter introduced are interpreted to include all such modifications, permutations, additions and sub-combinations as are within their true spirit and scope.

## Claims

1. A product of p-tert-butylphenyl propionaldehyde and m-tert-butylphenyl propionaldehyde, the product comprising m-tert-butylphenyl propionaldehyde in an amount from 6% to 60% of the combination of p-tert-butylphenyl propionaldehyde and m-tert-butylphenyl propionaldehyde.

2. A product according to claim 1 wherein the product comprises m-tert-butylphenyl propionaldehyde in an amount from one of 30% to 60% and 50% to 55% of the combination of p-tert-butylphenyl propionaldehyde and m-tert-butylphenyl propionaldehyde.

3. A product according to claim 1 obtained by:
adding dropwise an aldehyde solution to a basic solution; and, collecting a fraction of p-tert-butyl cinnamic aldehyde m-tert-butyl cinnamic aldehyde therefrom; and,
adding a transition metal catalyst to a solution of the p-tert-butyl cinnamic aldehyde in ethanol, and, collecting therefrom by distillation a product of p-tert-butylphenyl propionaldehyde and the meta isomer m-tert-butylphenyl propionaldehyde; wherein the product comprises m-tert-butylphenyl propionaldehyde in an amount from 6% to 60% of the combination of p-tert-butylphenyl propionaldehyde and m-tert-butylphenyl propionaldehyde.

4. A method for the production of p-tert-butylphenyl propionaldehyde and the m- isomer m-tert-butylphenyl propionaldehyde, the method comprising:
adding an aldehyde solution to a basic solution; and, collecting a fraction of p-tert-butyl cinnamic aldehyde and m-tert-butyl cinnamic aldehyde therefrom; and,
adding a transition metal catalyst to a solution of the p-tert-butyl cinnamic aldehyde in a solvent, and, collecting therefrom a product of p-tert-butylphenyl propionaldehyde and the meta isomer m-tert-butylphenyl propionaldehyde; wherein the product comprises m-tert-butylphenyl propionaldehyde in an amount from 6% to 60% of the combination of p-tert-butylphenyl propionaldehyde and m-tert-butylphenyl propionaldehyde.

5. A method according to claim 4 further including:
adding an acid to a solution of p-tert-butyltoluene to generate an m-isomer;
oxidizing the resulting mixture to yield p-tert-butylphenyl benzaldehyde and m-tert-butylphenyl benzaldehyde.

6. A method according to claim 4 wherein the catalyst includes a transition metal cataylyst selected from Ni, Pd/C and a Pd-Ni composite.

7. A method according to claim 4 wherein the basic solution includes one or more of a solvent selected from ethanol, water, and a mixture of ethanol and water.

8. A method according to claim 5, wherein the steps of
adding an acid to a solution of p-tert-butyltoluene to generate an m-isomer;
oxidizing the resulting mixture to yield p-tert-butylphenyl benzaldehyde and m-tert-butylphenyl benzaldehyde; are preliminary to the step of:
adding to an aldehyde solution to a basic solution which step also includes adding the aldehyde and basic solutions to the resulting oxidized mixture; and then collecting p-tert-butylcinnamic aldehyde and m-tert-butylcinnamic aldehyde therefrom;.

9. A method according to claim 8 wherein the product comprises m-tert-butylphenyl propionaldehyde in an amount from 6% to 60% of the combination of p-isomer and m-isomer.

10. A method according to claim 8 wherein the product comprises m-tert-butylphenyl propionaldehyde in an amount from 30% to 60% of the combination of p-isomer and m-isomer.

11. A method according to claim 8 wherein the product comprises m-tert-butylphenyl propionaldehyde in an amount from 50% to 55% of the combination of p-isomer and m-isomer.

12. A method according to claim 8 wherein the basic solution of the third step includes a base selected from zinc oxide, NaOH, KOH, Na2CO3 and K2C03.

13. A method according to claim 8 wherein the basic solution of the third step includes a solvent of a mixture of ethanol and water and a basic catalyst of NaOH dissolved therein.

14. A method according to claim 8 wherein the basic solution of the third step includes an aldehyde solution including substantially pure aldehyde (≥98%) or aldehyde in water.

15. A method according to claim 8 wherein the basic solution of the third step further includes a neutralization to about pH 7-8 by addition of HCl after the adding of the aldehyde solution.
